# EUROPEAN PATENT APPLICATION

(11) **EP 2 011 542 A2**
(43) Date of publication of application: **07.01.2009**
(21) Application number: 08252195.6
(22) Date of filing: 26.06.2008
(51) Int. Cl.: A61Q 1/10, A61Q 1/12, A61Q 3/02, A61Q 5/06, A61Q 5/12, A61Q 17/04, A61Q 19/08, A61K 8/02, A61K 8/58

(54) **Cosmetic composition comprising fluidized-particles**

(30) Priority: 27.06.2007 US 946566
(71) Applicant: L'Oreal, 75008 Paris (FR)
(72) Inventor: Quadir, Murat, Scotch Plains, 07076 New Jersey (US); Schultze, Xavier, 77340 Pontault-Combault (FR); Genain, Gilles, 75116 Paris (FR)
(74) Representative: Rutherford, Jodie

(57) **Abstract**

Cosmetic compositions for caring for and/or making up the human body comprising fluidized-particles are disclosed. Fluidized-particles have a solid core that is modified to exhibit liquid-like properties in the absence of solvent. In particular, the disclosure relates to the use of fluidized-particles in lipsticks, eyeliners, face and body creams, eye shadows, foundations, sun-protection products, deodorants, and shampoos, hair styling and leave-in hair treatments such as gels, mousses, sprays, conditioners, all of which may be in the form of an aqueous gel, lotion, or cream, or cast as a stick or as a disk.

## Description

### TECHNICAL FIELD

The present disclosure relates to cosmetic compositions for caring for and/or making up the human body comprising fluidized-particles. Fluidized-particles have a solid core that is modified to exhibit liquid-like properties in the absence of solvent. In particular, the disclosure relates to the use of fluidized-particles in lipsticks, eyeliners, face and body creams, eye shadows, foundations, sun-protection products, tanning products, deodorants, and shampoos, hair styling and leave-in hair treatments' such as gels, mousses, sprays, conditioners, all of which may be in the form of an aqueous gel, lotion, or cream, or cast as a stick or as a disk.

### BACKGROUND

Cosmetic compositions contain a variety of ingredients such as solvents, additives, minerals, coloring agents, etc. As long as these ingredients are liquid and/or soluble in the cosmetic medium, the preparation of the cosmetic composition does not present any problems. However, the incorporation of solid and/or insoluble ingredients presents formulation challenges and can affect the stability of the composition. It is essential to provide formulations which are homogeneous and do not quickly lose their cosmetic properties during use or do not leave sediment or phase separate upon prolonged storage. Gums, thickeners and emulsions can be used to help overcome these problems but the results are often unsatisfactory. For example, the viscosity may be too high or the appearance and texture too grainy. Volatile solvents may be used to increase shine and glossiness but as the solvents evaporate, the user is left with a feeling of dryness.

### BRIEF SUMMARY OF THE DISCLOSURE

The present disclosure relates to cosmetic compositions for caring for and/or making up the human body comprising fluidized-particles. Specifically, the disclosure relates to the use of fluidized-particles in lipsticks, eyeliners, face and body creams, eye shadows, foundations, sun-protection products, tanning products, deodorants, and shampoos, hair styling and leave-in hair treatments such as gels, mousses, sprays, conditioners, all of which may be in the form of an aqueous gel, lotion, or cream, or cast as a stick or as a disk. In certain aspects of the disclosure, the fluidized particles function to improve texture, shine, and adhesive properties. The fluidized particles may additionally or alternatively act as coloring agents, preservatives, surfactants, or solubilizing agents. Due to their liquid-like properties, the fluidized particles may be used in the manufacture of anhydrous cosmetics.

The fluidized-particles have a core, which is a solid below about 200°C at atmospheric pressure and having an average particle size of about 1x10⁻³ microns to about 5 microns. One or more carbon-containing tails attach to the core such that the resulting compound has about 50% to about 85% by weight organic content. Often, the fluidized-particles are salts and are unique in exhibiting liquid-like properties in the absence of solvent. The cosmetic compositions of the present disclosure generally comprise a positive amount up to about 85% of fluidized-particles, or from about 0.1% to about 85% of fluidized-particles.

The carbon-containing tails may comprise alkylamine moieties. For instance, the carbon-containing tails can be derived from organosilanes or sulfides. In one embodiment, the carbon-containing tails are PEG-containing quaternary ammonium cations. In another embodiment, the carbon-containing tail has the following formula: 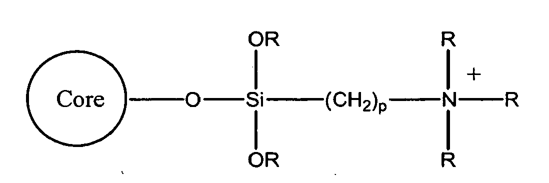 wherein:
p is an integer between 1 and 15 inclusive;
each R is independently a C₁-C₂₀ straight or branched alkyl. For instance, the carbon-containing tail may be -O-Si-(CH₃O)₂(CH₂)₃N⁺(CH₃)(C₁₀H₂₁)₂.

In another embodiment, the carbon-containing tail has the following formula: 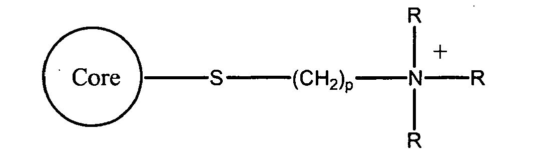 wherein:
p is an integer between 1 and 15 inclusive;
each R is independently a C₁-C₂₀ straight or branched alkyl. For instance, the carbon-containing tail may be -S-(CH₂)₃N⁺(CH₃)(C₈H₁₆)₂.

The counter-ion typically has a molecular weight of at least about 200 and may comprise polyoxyethylene. In some instances, the counter-ion further comprises a sulfonate. In one embodiment, the counter-ion is isosterate, oleate or has the following structure: 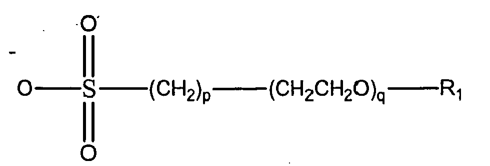 wherein:
p is an integer between 1 and 15 inclusive;
q is an integer between 1 and 30 inclusive; and
R₁ is a C₁-C₄₀ carbon moiety. For instance, the counter-ion is C₁₃-C₁₅ alkyl(OCH₂CH₂) ₇O(CH₂)₃SO₃⁻ or C₁-C₁₅ alkyl-phenyl-(OCH₂CH₂) ₂₁(CH₂)SO₃⁻.

The core may comprise a metal, a transition metal, or a polyoxometalate. For instance, the core may comprise platinum, gold, palladium, rhodium, SiO₂, TiO₂, ZnO, γ-Fe₂O₃, or H₃PW₁₂O₄₀. Typically, the core is a solid below temperatures of about 200°C and has an average particle size of about 1 × 10⁻³ microns to about 5 microns.

In one embodiment, the fluidized-particles have a core comprising H₃PW₁₂O₄₀ (12-tungsto phosphoric acid). For instance, the fluidized-particles are represented by the following formula: H₃₋ₓSₓPW₁₂O₄₀ where x is about 2 and S is (CH₃)(C₁₈H₃₇)N⁺[(CH₂CH₂O)ₙH][(CH₂CH₂O)ₘH] where n+m is 15. In another embodiment the fluidized-particles are salts of the reaction product of a heteropolyacid (such as polyoxymetalate) and a polyoxyethylene (such as polyoxyethlyene(15)octadecylamine).

Also provided are methods of applying fluidized-particles to keratinous areas of the body comprising applying a cosmetic composition comprising a fluidized-particle. The keratinous areas of the body may include, for example, skin, hair, nails, face, lips, and eyelashes.

### BRIEF DESCRIPTION OF THE DRAWINGS

The disclosure is best understood from the following detailed description when read in connection with the accompanying drawings. It is emphasized that, according to common practice, the various features of the drawings are not to scale. On the contrary the dimensions of the various features are arbitrarily expanded or reduced for clarity. Included in the drawings are the following figures:

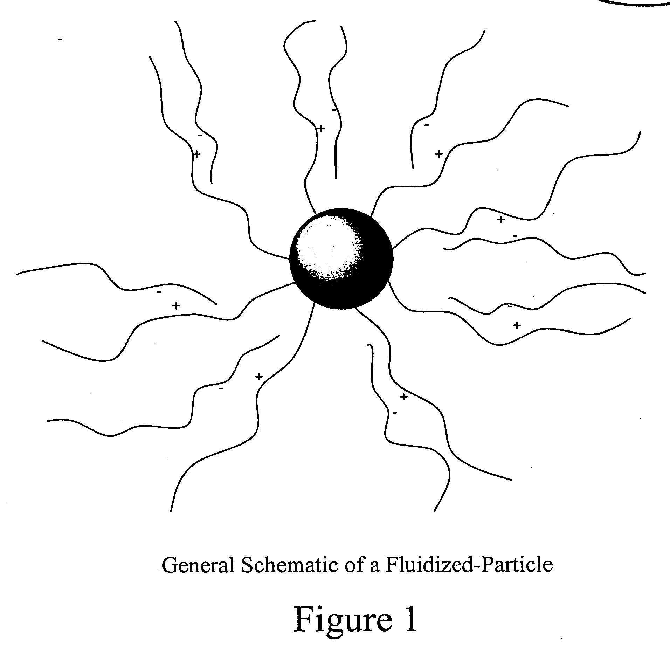

FIG. 1 is a general schematic of a fluidized-particle having a core, positively charged organic moieties attached thereto and counter-ions.

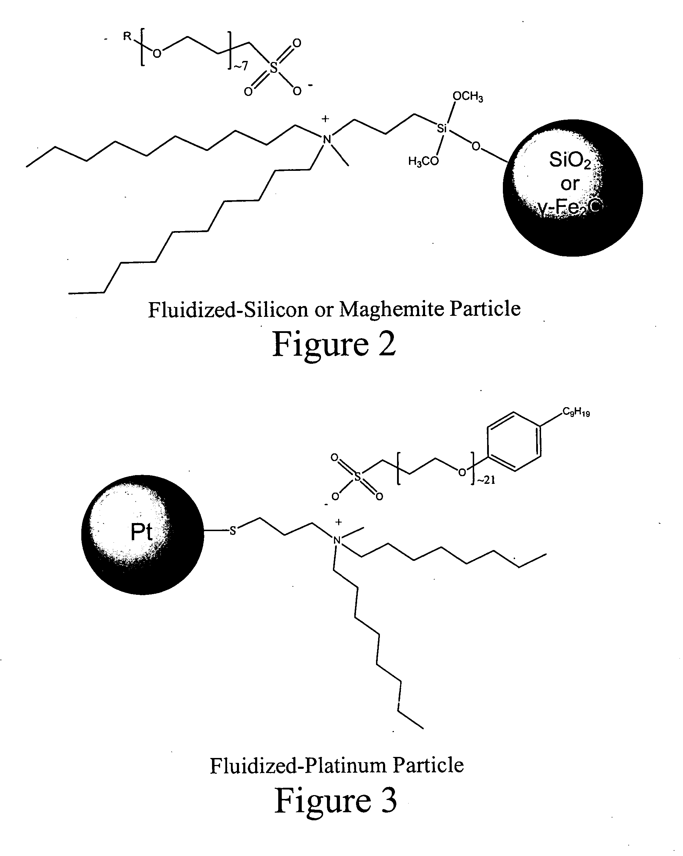

FIG. 2 is a schematic of a silicon or maghemite particle.
FIG. 3 is a schematic of a fluidized-platinum particle.

### DETAILED DESCRIPTION OF THE DISCOSURE

The present disclosure is drawn to cosmetic compositions comprising fluidized-particles. The fluidized-particles have a core, which is a solid below about 200°C at atmospheric pressure and have an average particle size of about 1x10⁻³ microns to about 5 microns. One or more carbon-containing tails attach to the core such that the resulting compound has about 50% to about 85% by weight organic content, for example, 55, 60, 65, 70, 75 or 80% by weight organic content. More particularly, the fluidized particles comprise a core, one or more carbon-containing tails and a counter ion. Often, the fluidized-particles are salts and are unique in exhibiting liquid-like properties in the absence of solvent. The cosmetic compositions of the present disclosure generally comprise a positive amount up to about 85% of fluidized-particles, or about 0.1 % up to about 85% fluidized-particles. The cosmetic composition may, for instance, comprise about 0.01% (or 0.02, 0.03, 0.04, 0.05, 0.2, 0.3, 0.4, 0.5, 0.6, 0.7, 0.8, 0.9%) to about 50% (or about 30, 35, 40 or 45%), or about 0.01% to about 25%, or about 0.01% to about 10% or about, or about 1% to about 10%, or about 1% to about 5% of fluidized-particles, for example 2, 3 or 4%.

As used herein, the term "demonstrates liquid-like behavior" or "exhibits liquid-like behavior" means that the substance appears or behaves like a liquid although it is physically in a solid state. More particularly, the phrases mean conductivity following the Vogel-Tammann-Fulcher (VTF) expression and shear loss modulus G" higher than the storage modulus G', e.g., at least one order of magnitude higher than the storage modulus. VTF expression is more fully explained in MacDonald et al., IMPEDANCE SPECTROSCOPY: EMPHASIZING SOLID MATERIALS AND SYSTEMS, 346 (Wiley, New York 1987), which is incorporated herein by reference in its entirety. The phrase, "absence of solvent" means generally an amount of solvent less than about 10% the weight of the fluidized particles. In other embodiments the fluidized particles exhibit liquid-like properties in the absence of solvent wherein the amount of solvent is less than about 5% or about 2% or about 1% or about 0.5% (or 0.4, 0.3, 0.2 or 0.1%) the weight of the fluidized particles.

The carbon-containing tails may comprise alkylamine moieties. For instance, the carbon-containing tails can be derived from organosilanes or sulfides. In one embodiment, the carbon-containing tails are PEG-containing quaternary ammonium cations. In another embodiment, the carbon-containing tail has the following formula: 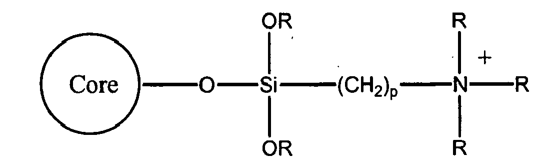 wherein:
p is an integer between 1 and 15 inclusive;
each R is independently a C₁-C₂₀ straight or branched alkyl. For instance, the carbon-containing tail may be -O-Si-(CH₃O)₂(CH₂)₃N⁺(CH₃)(C₁₀H₂₁)₂.

In another embodiment, the carbon-containing tail has the following formula: 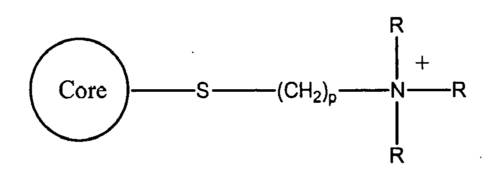 wherein:
p is an integer between 1 and 15 inclusive;
each R is independently a C₁-C₂₀ straight or branched alkyl. For instance, the carbon-containing tail may be -S-(CH₂)₃N⁺(CH₃)(C₈H₁₆)₂.

The counter-ion typically has a molecular weight of at least 200 and may comprise polyoxyethylene. In some instances, the counter-ion further comprises a sulfonate. In one embodiment, the counter-ion is isosterate, oleate or has the following structure: 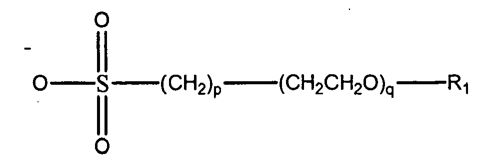 wherein:
p is an integer between 1 and 15 inclusive;
q is an integer between 1 and 30 inclusive; and
R₁ is a C₁-C₄₀ carbon moiety. In one embodiment, the counter-ion is C₁₃-C₁₅ alkyl(OCH₂CH₂) ₇O(CH₂)₃SO₃⁻ or C₁-C₁₅ alkyl-phenyl-(OCH₂CH₂)₂₁(CH₂)SO₃⁻.

The core may comprise a metal, a transition metal, or be a polyoxometalate. For instance, the core may be platinum, gold, palladium, rhodium, SiO₂, TiO₂, ZnO, γ-Fe₂O₃, or H₃PW₁₂O₄₀. Typically, the core is a solid below temperatures of about 200°C and has an average particle size of about 1x10⁻³ microns to about 5 microns microns, from about 1x10⁻³ microns to about 3 microns, from about 1x10⁻³ to about 1 micron, from about 1x10⁻³ to about 1 micron, from about 1x10⁻³ to about 0.25 microns, from about 1x10⁻³ to about 0.25 microns, from about 1x10⁻³ to about 0.1 microns, or from about 1x10⁻³ to about 2.5x10⁻² microns. In one embodiment, the fluidized-particle comprises a quaternary ammonium derivative of H₃PW₁₂O₄₀ (12-tungsto phosphoric acid). For instance, the fluidized-particles are represented by the following formula: H₃₋ₓSₓPW₁₂O₄₀ where x is about 2 and S is (CH₃)(C₁₈H₃₇)N⁺[(CH₂CH₂O)ₙH][(CH₂CH₂O)ₘH] where n+m is 15. In another embodiment the fluidized-particles are a salts of the reaction product of a heteropolyacid (such as polyoxymetalate) and a polyoxyethylene (such as polyoxyethlyene(15)octadecylamine).

Also provided are methods of applying fluidized-particles to keratinous areas of the body comprising applying a cosmetic composition comprising a fluidized-particle. The keratinous areas of the body may include, for example, skin, hair, nails, face, lips, and eyelashes.

The cosmetic compositions therefore can be used to alter the colour of skin, hair or nails or to alter their appearance or to improve the condition (e.g. to improve manageability or feel) of hair, nails or skin. The invention encompasses methods for altering hair, skin or nail colour comprising the step of applying a cosmetic composition of the present invention to hair, skin or nail. Further, methods for improving the condition of hair, skin or nail are encompassed using the cosmetic composition of the invention.

Methods for preparing fluidized-particles are disclosed in the following examples and also provided in International Patent Application Publication No. W02006/110166 filed on August 31, 2005 by Cornell Research Foundation, Inc., Bourlinos et al., ADV. MATER. 17(2):234-237 (2005) Warren et al., POLYMER PREPRINTS 47(2): 858-859 (2006), Trasbelsi et al., ADV. MATER. 18:2403-2406 (2006), Warren et al., J. AM. CHEM. SOC., 128:12074-12075 (2006), Bourlinos et al., SMALL 2(4):513-516, Bourlinos et al., EUR. PHYS. J., E20:109-117 (2006) and Bourlinos et al, ADV. FUNCT. MATER. 15:1285-1290 (2005), all of which are incorporated herein by reference in their entirety.

The fluidized-particles of the present disclosure are combined with one or more cosmetic or dermatological agents. For example, cosmetic compositions include a face powder, an eye shadow, a concealer, a lip composition, an eyeliner, a mascara, a nail paint or varnish, a care base, a fixing base for the lips, a dermatological or care product for the skin or keratinous fibers, a composition for protection against the sun or for artificial tanning, a product for cleansing the skin or keratinous fibers, a deodorant product, or a screening product. The cosmetic compositions may further include coloring agents, preservatives, antioxidants, fragrances, surfactants, and mixtures thereof.

Aspects of the present disclosure are illustrated in the following non-limiting examples.

### Example 1

### Fluidized-H₃₋ₓS_{X}PW₁₂O₄₀ Particles

5 grams of solid hereopolyacid H₃PW₁₂O₄₀ (12-tungsto phosphoric acid) is treated with 5 ml of (CH₃)(C₁₈H₃₇)N⁺[(CH₂CH₂O)ₙH][(CH₂CH₂O)ₘH] Cl⁻ (m+n=15) at 75°C for 2 hours. The solid acid is gradually dissolved by simultaneous evolution of gas according to the reaction: H₃POM + x S⁺Cl⁻ → H₃₋ₓSₓPOM = x HCl, ("POM" = polyoxometalate) where S⁺ stands for the polyethylene glycol ("PEG") containing ammonium cation. The clear liquid is extracted several times with a hot toluene/acetone mixture (85:15 v/v) to remove any residual solvent. Finally, the remaining product is carefully washed with water and re-dried at 75°C for 24 hours to yield fluidized-H₃₋ₓS_{X}PW₁₂O₄₀ being a viscous, optically transparent liquid at room temperature.

### Example 2

### FIuidized-H₃₋ₓS_{X}PW₁₂O₄₀ Particle Salts

5 grams of solid heteropolyacid ("POM") of example 1 are treated with polyoxyethylene(15)octadecylamine, 2 amine moles per mole of POM in water. After homogenization, the solvent is allowed to evaporate at room temperature. The product is the salt reaction product of POM and polyoxyethylen(15)octadecylamine and is a liquid at room temperature.

### Example 3

### Fluidized-Silica Particles

3.5 mL of colloidal silica (particle size: 7x10⁻³ microns, pH=10) is diluted with 20 mL de-ionized water. 5 mL of (CH₃O)₃Si(CH₂)₃N⁺(CH₃)(C₁₀H₂₁)₂ Cl⁻ in methanol (40%) are added to the suspension. A white precipitate immediately forms and is aged for 24 hours at room temperature by gently shaking from time to time. The solvent is discarded and the solid is rinsed three times with water and twice with ethanol. The solid is re-suspended in ethanol, poured into a Petri dish and dried at 70°C. The corresponding sulfanate salt is prepared by treating 1 gram of the chloride analog with 15mL of a 10.5 w/v % solution of R(OCH₂CH₂)₇O(CH₂)₃SO₃⁻K⁺ (R:C₁₃-C₁₅ alkyl chain) in water at 70°C for 24 hours. Following removal of the solution, the material is washed several times with water and dried at 70°C. The isosterate and oleate salts are prepared similarly by ion-exchanging the chloride with isosterate and oleate salts except using 6.6 w/v % solution of carboxylate salt.

### Example 4

### Fluidized-Maghemite Particles

Maghemite particles (γ-Fe₂O₃) are prepared by adding 5mL of concentrated ammonia (28-30%) to 15mL of ethanol solution containing 6 grams of Fe(NO₃)₃·9H₂0 under vigorous stirring. The as-formed iron hydroxide precipitate is centrifuged, washed well with water and air-dried. 2 grams of the freshly prepared iron hydroxide is suspended in 40mL ethylene glycol and the suspension is purged with flowing argon prior to refluxing under an argon atmosphere for 1 hour. After reaction, the admixture is cooled to room temperature, centrifuged and washed with acetone, then water. The solid is re-suspended in acetone and air-dried.

The fluidized-maghemite particles are prepared by dispersing 0.5 grams of γ-Fe₂O₃ in 10mL of alkaline H₂O (pH= 10, NH₄OH) and sonicating the suspension for 25 minutes in a warm bath followed by the addition of 3mL of the silane solution. The precipitate is aged for 24 hours at room temperature, filtered and washed repeatedly with water and methanol, then dried. The dried solid is dissolved in tetrahydrofuran and left undisturbed for 10 minutes to allow any insoluble particles to settle. The clear brown supernatant liquid is evaporated to leave behind the maghemite chloride salt. The sulfonate derivative is prepared similarly to the silica analog by ion exchange with R(OCH₂CH₂)₇O(CH₂)₃SO₃⁻K⁺. After exchange, a waxy brown material is obtained, washed with water, and dried at 70°C to produce a viscous liquid.

### Example 5

### Fluidized-Platinum Particles

Excess 1,3-dibromopropane and N,N-dioctyl-N-methylamine is combined in a 30:1 molar ratio in air. Heating at 50°C for 12 hours followed by vacuum distillation of the excess 1,3-dibromopropane and followed by exposing the sample to high vacuum overnight at room temperature yields N-(3-bromopropyl)-N,N-dioctyl-N-methylammonium bromide.

In a nitrogen glove box, N-(3-bromopropyl)-N,N-dioctyl-N-methylammonium bromide is dissolved in five times its mass of anhydrous, degassed methanol. Sodium hydrosulfide hydrate is added in five equal portions with a three hour gap between additions of each portion. Methanol is removed by vacuum and then the dissolution of N,N-dioctyl-N-(3-mercaptopropyl)-N-methylammonium bromide in chloroform precipitates sodium bromide. The chloroform is removed under vacuum.

300 mg of H₂PtCl₆·6H₂O, 225 grams of N,N-dioctyl-N-(3-mercaptopropyl)-N-methylammonium bromide are stirred in 20mL of anhydrous tetrahydrofuran ("THF") for thirty minutes. Subsequently, 10mL of 1.0 M superhydride in THF is injected at a constant rate over about 10 seconds. After stirring for about 30 minutes, a small amount of methanol is added to quench any excess superhydride. Rotary evaporation to remove THF followed by three cycles of centrifugation in methane:water (20:1) affords the pure platinum particles.

The bromide anion on the platinum particle is exchanged with a bulky sulfonate anion by addition of a stoichiometric amount of poly(ethyleneglycol) 4-nonylphenyl 3-sulfopropyl ether potassium salt in chloroform. The reaction is stirred for 24 hours, after which an equal volume of water is added and the reaction is stirred for another 24 hours. The water is removed in a separatory funnel and the chloroform is rotary evaporated. All the solvents are removed by exposure to high vacuum overnight to yield the final fluidized-platinum particles.

### Example 6

### Fluidized-Zinc Oxide

Fluidized-Zinc Oxide is prepared by dispersing about 0.5 grams of ZnO in about 10mL of ethanol. The mixture is sonicated for 5 minutes followed by the addition of three drops of NaOH aqueous solution (10% w.v). The dispersion is sonicated another 5 min. before (CH₃O)₃Si(CH₂)₃N⁺(CH₃)(C₁₀H₂₁)₂Cl⁻ in 3mL of methanol (40%) is added. After an additional 5 min. of sonication, 5mL of H₂O is added to the mixture, and the final composition sonicated for another 5 min. During this time a bulk precipitate is formed. The precipitate is aged for 24 hours at room temperature, rinsed with water and methanol, and dried. The dried solid is dispersed in tetrahydrofuran ("THF") and left undisturbed for 5 min. to settle any undispersed particles. The supernatant dispersion is collected and evaporated. A sulfonate salt is prepared by treating the chloride analogue with 15mL of a 16.5% (w/v) solution of C₉H₁₉-C₆H₄-(OCH₂CH₂)₂₀O(CH₂)₃SO₃⁻K⁺ (poly(ethylene glycol) 4-nonylphenyl 3-sulfopropyl ether, potassium salt) in water at 75°C for 24 hours. The resultant dispersion is treated with toluene to extract any excess of the sulfonate salt. The aqueous dispersion is evaporated in a steam bath and then redispersed in 15mL of H₂O. Any remaining sulfonate salt is extracted several times with 30mL portions of warm toluene. The aqueous layer is collected and evaporated in a steam bath. The remaining material is dispersed in about 15mL of acetone and the clear sol obtained after centrifuging is air-dried at 65°C for three days and then vacuum-dried at room temperature for another day.

### Example 7

| Hair Spray | |
|---|---|
| **Ingredient** | **Percentage (%)** |
| Fluidized-SiO₂, γ-Fe₂O₃,ZnO or TiO₂ | 3% |
| Ethanol | 10% |
| Dimethyl Ether | 35% |
| Water | q.s. to 100% |

3g of Fluidized-SiO₂, γ-Fe₂O₃ or Pt is dispersed in 10g Ethanol and 52g of Water. This dispersion is pressurized with 35g of Dimethyl Ether.

### Example 8

| Hair Spray | |
|---|---|
| **Ingredient** | **Percentage (%)** |
| Fluidized-Pt, Au, Pd, or Rh | 3% |
| Water | q.s. to 100% |

For Zero-VOC applications, 3g of Fluidized-SiO₂, γ-Fe₂O₃ or Pt is dispersed in 97g of water. The dispersion is pressurized between 9 and 11 bars with air.

### Example 9

| Hair Mousse | |
|---|---|
| **Ingredient** | **Percentage (%)** |
| Fluidized-SiO₂, γ-Fe₂O₃,ZnO, TiO₂, Pt, Au, Pd, or Rh | 2% |
| Empicol BSD 52 | 1.5% |
| Propellant A-46 | 6% |
| Water | q.s. to 100% |

### Example 10

| Hair Gel | |
|---|---|
| **Ingredient** | **Percentage (%)** |
| Fluidized- SiO₂, γ-Fe₂O₃, ZnO, TiO₂, Pt, Au, Pd, or Rh | 5% |
| Acrylates/C₁₀-C₃₀ alkyl | 0.5% |
| Acrylate Crosspolymer PVP | 3% |
| Water | q.s. to 100% |

### Example 11

| Leave-In Conditioner | |
|---|---|
| **Ingredient** | **Percentage (%)** |
| Fluidized- SiO₂, γ-Fe₂O₃, ZnO, TiO₂, Pt, Au, Pd, or Rh | 3% |
| Behentrimonium Chloride | 1% |
| Cetearyl Alcohol | 5% |
| Cetyl Esters | 1% |
| Dow Corning 939 Emulsion | 4% |
| Water | q.s. to 100% |

### Example 12

| Anti-Aging Cream | |
|---|---|
| **Ingredient** | **Percentage (%)** |
| Fluidized- SiO₂, γ-Fe₂O₃, ZnO, TiO₂, Pt, Au, Pd, or Rh | 3% |
| Pemulen^{™} TR1 | 2% |
| Water | q.s. to 100% |

3g of Fluidized-SiO₂, γ-Fe₂O₃ or Pt is dispersed in 95g of water and 2g of Pemulen^{™} TR1 (as thickener).

### Example 13

| | | |
|---|---|---|
| Foundation | | |
| | **Ingredient** | **Percentage (%)** |
| Phase A | Cyclopentasiloxane/Dimethicone Copolyol | 8% |
| | Polyglyceryl-4-isostearate/Hexyl laurate/Cetyl PEG/PPG-10/ 1-Dimethicone | 3.5% |
| | Pigments | 10% |
| Phase B | Fluidized- SiO₂, γ-Fe₂O₃, ZnO, TiO₂, Pt, Au, Pd, or Rh | 6% |
| Phase C | Preservative | 0.4% |
| | Disteardimethimonium Hectorite | 0.6% |
| | Propylene carbonate | 0.2% |
| Phase D | Magnesium Sulfate | 1% |
| | Preservative | 0.7% |
| | Non ionic emulsifier | 0.5% |
| | Water | q.s. to 100% |

### Example 14

| Sunscreen | | |
|---|---|---|
| | **Ingredient** | **Percentage (%)** |
| Phase A | Water | 31.4% |
| | Avicel RC 591 (microcrystalline cellulose (and) cellulose gum) | 1.5% |
| | Rhodicare S 2% Solution (xanthan gum) | 20% |
| | Disodium EDTA | 0.1% |
| Phase B | DC 200 Fluid 350 CS (dimethicone) | 6% |
| | Cetyl Alcohol | 2.5% |
| | Isostearate Isostearyle (Gattefossé) | 5% |
| | Myritol 331 (cocoglycerides) | 4% |
| | Labrafac CC (Gattefossé) (caprylic/capric triglycerides) | 8.5% |
| | Parsol MCX (Roche) (octyl methoxycinnamate) | 6% |
| | Parsol 1789 (Roche) (butyl dibenzoylmethane) | 1.5% |
| | Eusolex 4360 (benzophenone-3) | 1.5% |
| | Vitamin E Acetate (tocopheryl acetate) | 0.5% |
| Phase C | Fluidized- SiO₂, γ-Fe₂O₃, ZnO, or TiO₂ | 5% |
| Phase D | Emulfree P (propylene glycol laurate (and ethylcellulose (and) propylene glycol isostearate) | 6% |
| Phase E | Gatuline Λ (pilewort extract) | 0.1% |
| | Glydant Plus Liquid (DMDM hydantoin (and) iodopropynyl butylcarbamate) | 0.4% |

The fluidized-particles used in sunscreen may have three phases including a UV filter encapsulated in one phase, moisturizer in a second phase, and an adhesive in a third phase.

### Example 15

| Mascara | |
|---|---|
| **Ingredient** | **Percentage (%)** |
| Fluidized- SiO₂, γ-Fe₂O₃, ZnO, TiO₂, Pt, Au, Pd, or Rh | 3% |
| Stearic acid | 6% |
| Beewax | 3% |
| Carnauba wax | 3% |
| Paraffin | 13% |
| Conditioning Agent | 6% |
| Panthenol | 0.5% |
| Water | q.s. to 100.00% |

### Example 16

| Nail Enamel | |
|---|---|
| **Ingredient** | **Percentage (%)** |
| Fluidized- SiO₂, γ-Fe₂O₃, ZnO, TiO₂, Pt, Au, Pd, or Rh | 4% |
| Cosmetically acceptable medium | 48% |
| Plasticizers | 18% |
| Acrylics | 4.5% |
| Nitrocellulose | 12% |
| Zeeospheres^{™} W210 | 0.5% |
| Bentone gel | 13% |

As used herein, the term "cosmetically acceptable" means that the compositions are suitable for cosmetic application to keratinous areas of the body. A "cosmetically acceptable carrier' as used herein includes any vehicle or base which is capable of delivering fluidized-particles to keratinous areas of the body for use as a cosmetic.

The cosmetic compositions of the present disclosure may include oils, which are liquid at room temperature, waxes or gums which are solid at room temperature, pasty fatty substances of animal, vegetable, mineral, or synthetic origin, their mixtures, and inorganic powders such as those disclosed herein, can be used in combination with fluidized-particles. There is no limitation on the additional fatty phase, and it can comprise products which are fluid at room temperature, such as silicone oils, fluorinated oils, fluorosilicone oils, or hydrocarbonaceous oils which are optionally partially siliconated. These oils may be volatile at room temperature and atmospheric pressure. These oils can represent a positive amount up to about 80% of the total weight of the composition or from about 0.1% to about 50%.

The cosmetic composition according to the present disclosure can comprise hydrocarbonaceous, fluorinated, or silicone waxes, or their mixtures, which can be solid or semisolid (i.e., in the form of a paste) at room temperature. These waxes can be of vegetable, mineral, animal, and/or synthetic origin. These waxes exhibit in particular a melting temperature of greater than about 250°C. Alternatively they exhibit a melting temperature of greater than about 45°C.

Silicone waxes can be waxes comprising a silicone structure and units with one or more pendant alkyl or alkoxy chains and/or one or more alkyl or alkoxy chains at the end of the silicone structure, these chains being linear or branched and comprising from 10 to 45 carbon atoms. These waxes are known respectively as alkyl dimethicones and alkoxy dimethicones. Furthermore, these alkyl chains can comprise one or more ester functional groups. Additional waxes which can be used in accordance with the present disclosure are waxes of animal origin such as lanolin or beeswax, waxes of vegetable origin such as carnauba or candelilla wax, waxes of mineral origin such as paraffin wax, lignite wax, microcrystalline waxes, ceresin, or ozocerite, synthetic waxes such as polyethylene waxes, and mixtures thereof.

The presence of waxes in the compositions of the present disclosure makes it possible to provide good mechanical strength, in particular when the composition is provided in the form of a stick. The composition can comprise a positive amount up to about 50% of the total weight of the composition of wax, or from about 0.1 % to about 30%. These fatty substances should be chosen appropriately by a person skilled in the art in order to prepare a composition having the desired properties, for example, consistency and/or texture.

Moreover, the cosmetic compositions of the present disclosure can comprise at least one additional ingredient conventionally used in the field, such as antioxidants, essential oils, preservatives, cosmetic or dermatological active principles, such as moisturizers (glycerol), vitamins, essential fatty acids, lipophilic sunscreens, fat-soluble polymers, in particular hydrocarbonaceous polymers such as polyalkylenes, gelling agents for the aqueous phase, gelling agents for the fatty phase, fragrances, surfactants, and mixtures thereof. These additional ingredients can be present in the composition according to the amounts conventionally used, for example, a positive amount up to about 20% of the total weight of the composition, or from about 0.1 to about 10% of the total weight of the composition.

The cosmetic compositions of the present disclosure may comprise, as an additional ingredient, one or more gelling agents for the aqueous phase. For instance, agents for the aqueous phase which can be used according to the present disclosure of water-soluble cellulose gelling agents such as hydroxyethylcellulose, methylcellulose, hydroxypropylcellulose, and carboxymethylcellulose, guar gum, quaternized guar gum, nonionic guar gums comprising C₁-C₆ hydroxyalkyl groups, xanthan, locust bean, scleroglucan, gellan, rhamsan, or karoya gums, alginates, maltodextrin, starch and its derivatives, hyaluronic acid and its salt, clays such as montmorillonites, hectorites, bentones, or laponites, polymers with a carboxyl group such as crosslinked polyacrylic acids which are at least partially neutralized, for example, "Carbopol" or "Carbomer" products from the company Goodrich (Carbomer 980, for example, neutralized with triethanolamine, abbreviated TEA), poly(glyceryl (meth)acrylate) polymers, polyvinylpyrrolidone, poly(vinyl alcohol), crosslinked acrylamide polymers and copolymers, crosslinked methacryloyloxyethyltrimethylammonium chloride homopolymers, associative polyurethanes, and mixtures thereof. In another embodiment, according to the present disclosure, the gelling agent for the aqueous phase is chosen from xanthan gum, clays (bentone or laponite), associative polyurethanes, cellulose thickeners, in particular hydroxyethyl cellulose, crosslinked polyacrylic acid which are at least partially neutralized, and mixtures thereof.

Cosmetic compositions according to the present disclosure can be provided in the form of a colored product, in particular for making up the skin, especially a foundation, a face powder, an eyeshadow, a mascara, an eyeliner, a concealer stick, or a nail varnish, or a lip composition for making up the lips, such as a lipstick. They can also be provided in uncolored form, optionally comprising cosmetic or dermatological active principles. They can then be used as a care base for the lips (for example, lip balms, protecting the lips from the cold and/or the sun and/or the wind), or a fixing base to be applied with a conventional lip composition. The fixing base then forms a protective film on the lip film of color, which limits the transfer thereof.

The cosmetic compositions of the present disclosure can also be provided in the form of a dermatological or cosmetic composition for treating or caring for the hair, skin (including the scalp), nails, or lips, or in the form of a composition for sun protection or artificial tanning, or in the form of a product for cleansing or removing makeup from the skin or keratinous fibers, a deodorant product, or a scenting product.

Additionally, the cosmetic composition of the present disclosure can comprise a coloring material comprising in particular a particulate phase which is generally presented in a positive amount up to about 60% of the total weight of the composition, or from about 0.1% to about 35%, and which can comprise pigments, pearlescent agents, fillers, or any mixture thereof, commonly used in cosmetic compositions, or colorants which are soluble in the medium, in particular water-soluble or fat-soluble colorants.

The term "pigment" refers to a white or colored, inorganic or organic substance, which are insoluble to the medium of the composition and intended to color and/or improve the opacity of the composition. The term "fillers" refers to inorganic or synthetic, and lamellar or nonlamellar particles. The term "pearlescent agents" refers to iridescent particles produced, in particularly, by certain mollusks in their shells, or else synthesized. The fillers and pearlescent agents are used to modify the texture of the composition and the matteness/gloss effect.

The pigments can be present in the cosmetic compositions in an amount ranging from a positive amount up to about 60% of the weight of the final composition, or from about 0.1% to about 60% of the final composition, or from about 4% to about 25%. Inorganic pigments can be used in the cosmetic compositions such as titanium, zirconium, or cerium oxides, zinc, iron, or chromium oxides, and ferric blue. Organic pigments which can be used in the cosmetic compositions include carbon black and barium, _strontium, calcium, or aluminum lakes, and mixtures thereof.

Pearlescent agents can be present in the cosmetic compositions in an amount ranging from a positive amount to about 20% of the total weight of the composition, or from about 0.1% to about 15% of the total weight of the composition. Pearlescent agents which can be used in the cosmetic compositions include mica covered with titanium oxide, with iron oxide, with natural pigment, or with bismuth oxychloride, such as colored titanium oxide-coated mica.

Fillers can be present in an amount ranging from a positive amount to about 35% of the total weight of the cosmetic compositions, or from about 5% to about 15% of the total weight of the cosmetic composition. Fillers include, for example, talc, mica, silica, kaolin, powders formed of Nylon^{®} (Orgasol^{®}, in particular, from Atochem) and of polyethylene, Teflon^{®}, starch, boron nitride, microspheres formed of copolymers, such as Expancel^{®}. (Nobel Industrie) of Polytrap^{®} (Dow Coming), and silicone rein microbeads (Tospearl^{®} from Toshiba, for example), and mixtures thereof.

Another subject of the disclosure is a process for increasing the freshness, matteness, stability, softness, viscosity of a cosmetic composition, and/or for decreasing the transfer of the composition, the process comprising introducing into the composition fluidized-particles as defined above.

The term "comprising" (and its grammatical variations) as used herein is used in the inclusive sense of "having" or "including" and not in the exclusive sense of "consisting only of." The terms "a" and "the" as used herein are understood to encompass the plural as well as the singular.

The foregoing description illustrates and describes the present disclosure. Additionally, the disclosure shows and describes only the preferred embodiments of the disclosure, but, as mentioned above, it is to be understood that it is capable of changes or modifications within the scope of the concept as expressed herein, commensurate with the above teachings and/or skill or knowledge of the relevant art. The described hereinabove are further intended to explain best modes known of practicing the disclosure and to enable others skilled in the art to utilize the disclosure in such, or other embodiments and with the various modifications required by the particular applications or uses disclosed herein. Accordingly, the description is not intended to limit the disclosure to the form disclosed herein. Also it is intended that the appended claims be construed to include alternative embodiments.

All publications, patents and patent applications cited in this specification are herein incorporated by reference, and for any and all purposes, as if each individual publication, patent or patent application were specifically and individually indicates to be incorporated by reference. In this case of inconsistencies, the present disclosure will prevail.

## Claims

1. A cosmetic composition comprising a cosmetically acceptable carrier and about 0.01 % to about 85% of fluidized-particles wherein the fluidized-particles have (1) a core, which is a solid below about 200°C at atmospheric pressure and has an average particle size of about 1x10⁻³ microns to about 5 microns; and (2) at least one carbon-containing tail attached to the core such that the resulting compound has about 50% to about 85% by weight organic content and exhibits liquid-like behavior in the absence of solvent.

2. The cosmetic composition of claim 1 wherein the fluidized-particles are salts.

3. The cosmetic composition of claim 1 or 2 wherein the carbon-containing tail comprises an alkylamine moiety.

4. The cosmetic composition of claim 3, wherein the carbon-containing tail is a organosilane or a sulfide and the counter-ions have a molecular weight greater than 200.

5. The cosmetic composition of claim 4, wherein the carbon-containing tail has the following structure: 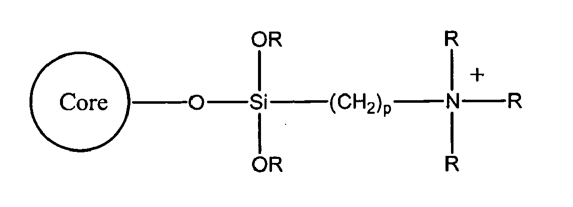 wherein:
p is an integer between 1 and 15 inclusive;
each R is independently a C₁-C₂₀ straight or branched alkyl or
wherein the carbon-containing tail has the following structure: 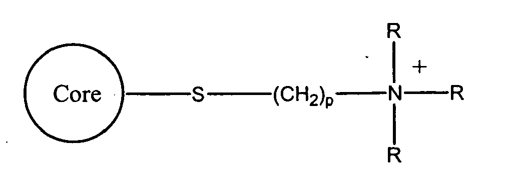 wherein:
p is an integer between 1 and 15 inclusive;
each R is independently a C₁-C₂₀ straight or branched alkyl.

6. The cosmetic composition of claim 5 wherein the carbon-containing tail is -O-Si-(CH₃O)₂(CH₂)₃N⁺(CH₃)(C₁₀H₂₁)₂ or the carbon-containing tail is -S-(CH₂)₃N⁺(CH₃)(C₈H₁₆)₂.

7. The cosmetic composition of any one of claims 2 to 6 wherein the counter-ion is isosterate, oleate or comprises polyoxyethylene.

8. The cosmetic composition of claim 7 wherein the counter-ion has the following structure: 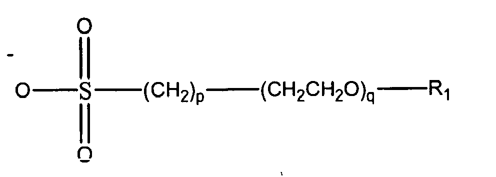 wherein:
p is an integer between 1 and 15 inclusive;
q is an integer between 1 and 30 inclusive; and
R₁ is a C₁-C₄₀ carbon moiety.

9. The cosmetic composition of claim 8 wherein the counter-ion is C₁₃-C₁₅ alkyl(OCH₂CH₂) ₇O(CH₂)₃SO₃⁻ or C₁-C₁₅ alkyl-phenyl-(OCH₂CH₂) ₂₁(CH₂)SO₃⁻.

10. A cosmetic composition comprising a cosmetically acceptable carrier and about 0.01 % to about 85% of fluidized-particles wherein the fluidized-particles comprise (1) a core having a diameter of about 1x10⁻³ microns to about 5 microns; and (2) at least one carbon-containing tail attached to the core, which is -O-Si-(CH₃O)₂(CH₂)₃N⁺(CH₃)(C₁₀H₂₁)₂; and a counter ion being C₁₃-C₁₅ alkyl(OCH₂CH₂)₇O(CH₂)₃SO₃⁻, or at least one carbon-containing tail attached to the core, which is -S-(CH₂)₃N⁺(CH₃)(C₈H₁₆)₂; and a counter ion being C₁-C₁₅ alkyl-phenyl-(OCH₂CH₂)₂₁(CH₂)SO₃⁻.

11. The cosmetic composition of claim 10 wherein the core is a polyoxometalate.

12. The cosmetic composition of claim 11 wherein the core is γ-Fe₂O₃ or SiO₂.

13. The cosmetic composition of claim 10 wherein the core is a metal.

14. The cosmetic composition of claim 13 wherein the core is selected from platinum, gold, palladium, and rhodium.

15. The cosmetic composition of claim 1 wherein the fluidized-particles are H₃₋ₓSₓPW₁₂O₄₀ where x is about 2 and S is (CH₃)(C₁₈H₃₇)N⁺[(CH₂CH₂O)ₙH][(CH₂CH₂O)ₘH] where n+m is 15.

16. A composition according to any one of claims 2 to 15, wherein the composition is a foundation, a face powder, an eye shadow, a concealer, a lip composition, an eyeliner, a mascara, a nail varnish, a care base, a fixing base for the lips, a dermatological or care product for the skin or keratinous fibers, a composition for anti-sun protection or artificial tanning, a product for cleansing the skin or keratinous fibers, a deodorant product, a hair and/or body shampoo products a hair and/or body conditioning product, or a hair styling product.

17. A composition according to any one of claims 2 to 15, wherein the fluidized particles function as a surfactant, preservative or preservative agent.

18. A composition according to claim 16, additionally comprising a coloring material and/or at least one ingredient chosen from preservatives, antioxidants, fragrances, surfactants, and mixtures thereof.

19. A method of applying a fluidized-particle to a keratinous area of the body, comprising applying a cosmetic composition according to any one of claims 1 to 15.

20. The method of claim 19 wherein the keratinous area of the body is selected from skin, hair, nails, face, lips, and eyelashes.
